# EUROPEAN PATENT APPLICATION

(11) **EP 1 764 046 A2**
(43) Date of publication of application: **21.03.2007**
(21) Application number: 06120390.7
(22) Date of filing: 08.09.2006
(51) Int. Cl.: A61B 17/16, A61B 17/00

(54) **Surgical shaping tool**

(30) Priority: 19.09.2005 GB 0519084
(71) Applicant: Finsbury (Development) Limited, Randalls Road Leatherhead, Surrey KT22 7BA (GB)
(72) Inventor: Taylor, Andrew Clive, Chichester, Surrey PO19 3QQ (GB); Tuke, Michael Antony, Guildford, Surrey GU1 3TF (GB)
(74) Representative: Smaggasgale, Gillian Helen

(57) **Abstract**

A surgical shaping tool (1) comprising: a body (2) having a leading end (5) and a drive end (4); a debris collecting store; at least one cutting means (6); and at least one means (7) for directing debris into the store.

## Description

The present invention relates to a tool for shaping and/or smoothing bores in hard tissue. More particularly, the present invention relates to a single use rasp for shaping and/or smoothing bores or recesses in hard tissue.

In many types of surgical procedure, it is necessary for bores, recesses, or other sites in hard tissue to be shaped and/or smoothed. The site may be artificially made by a surgeon or may be pre-existing. One example of a pre-existing bore which may have to be shaped and/or smoothed during surgery is the intramedullary canal in the femur which may require modification during hip replacement surgery before a femoral implant can be inserted. An example of a pre-existing recess which may have to be shaped and/or smoothed is the acetabulum.

Hip replacement operations are one of the most common operations carried out, with over 700,000 procedures being performed in Europe every year. During the operation, the surgeon accesses the hip joint and dislocates it before removing the femoral head to reveal the intramedullary canal. The entrance to the canal may be drilled to make the canal more accessible. The next step is the preparation of the femoral implant insertion site and this is achieved by using a broach or tamp, which is driven into the canal with a surgical mallet. It is very important that the surgeon's mallet blows are accurate to prevent unwanted stresses within the femur.

A broach functions by cutting material away from the surface of the canal to provide a bore which has a shape that corresponds to the shape of the femoral implant. A tamp is generally smooth and functions to shape the canal by deforming and impacting the material into the walls of the bore.

While the use of such tools to obtain a bore of the desired dimensions is well known, there are a number of drawbacks associated with their use. When broaches are used, their cutting faces soon become clogged with material which has been displaced from the bone during the operation of the broach. This causes the broach to function like a tamp, compacting tissue against the inner wall of the cortical bone.

This impaction of bone and other tissues into the walls of the bore whether by a tamp or by a clogged broach creates a high degree of stress within the femur which may cause intra-operative fractures in the femur. This problem is exacerbated by the additional stress caused by the mallet blows. It is estimated that intra-operative fractures of the femur occur in around 17% of all hip-replacement procedures.

While these fractures can be rectified intra-operatively, they may be overlooked and grow over time until post-operative surgery is required to correct the problem. In addition, fractures also occur post-operatively due to the stresses placed on the femur by the implant and its fixings. Fracturing of the ipsilateral femur after hip replacement surgery is a serious problem which is thought to cause around 2.3% of all post-operative femoral fractures.

In addition to conventional broaches and tamps, re-usable rasps have also been suggested to shape and smooth the intramedullary canal. However, as these too are impacted with a surgical mallet, the same unwanted stresses are applied to the femur as with other canal shaping devices. In addition, as with the broaches, the known rasps are prone to becoming clogged and when this occurs, they too function like tamps, which increases unwanted stress in the bone.

In addition to the unwanted mechanical stresses which occur when the intramedullary canal is drilled and shaped with conventional tools, heat is generated. This heat generation has the effect of killing tissue adjacent to the canal, which retards osteo-integration between the femoral prosthesis and the femur. Further, soft tissue may grow between the prosthesis and the femur at the site of tissue death which retards osteo-integration and causes patient discomfort.

It has also been suggested that the use of conventional shaping tools may be associated with the generation of fatty tissue embolisms. This is thought to be because when the loose material within the canal is impacted against the wall of the canal, this squeezes fat cells forcing nanoscopic amounts of fatty tissue out of the cell. Simultaneously, blood vessels are also ruptured, which allows the fatty tissues to mix with blood cells. Further compaction of this mixture forces it into the blood stream where the fatty tissue congeals and clots to form emboli. These emboli may then travel to the lungs where they can cause blockages in the pulmonary arteries. This prevents blood from nourishing the lung which may cause the tissues downstream from the blockage to be starved of oxygen, resulting in damage to the lung, and in extreme cases, to the death of the patient.

A further drawback associated with the use of conventional bore shaping devices relates to the re-use of surgical tools. Surgical tools are usually re-used with a sterilisation procedure being carried out between operations. This use/sterilisation cycle only concludes when the surgeon decides that the tool is failing to perform adequately. Worryingly, tests have shown that following sterilisation, microbes can still be present from previous operations, and these can include MRSA, VRSA, Hepatitis-B, CJD and other antibiotic resistant microbes. It is thought that the presence of such microbes may have been responsible for the deaths of up to 3144 patients in Europe (Department of Health 'Information about Infectious Diseases'). The same study also found that inadequate sterilisation procedures are carried out in approximately 43% of all hospitals resulting in instruments remaining contaminated with harmful bacteria.

In recent years, other surgical techniques for removing tissue have been developed. The use of lasers has been suggested as their use offers certain advantages, such as excellent haemostasis, relative absence of scarring and wound contraction, minimal damage to adjacent tissue and reduced post-operative swelling, pain and infection. However, lasers have limited effectiveness in hard tissues and additionally generate substantial heat in the femur which will damage adjacent tissues thus retarding osteo-integration. In addition, the beams from many of the lasers used in surgery are not visible to the eye making it difficult for the surgeon to ensure that surrounding tissue is not damaged.

Thus, it is desirable to provide a shaping tool which is capable of removing unwanted tissue from hard tissue without impacting said tissue into the wall of the operation site. It is also desirable to provide a shaping tool which does not easily become clogged with loosened material. It is further desirable to provide a shaping tool which reduces the number of stress fractures formed during surgical procedure. In addition, it is desirable to provide a shaping tool which does not damage tissue adjacent to the site at which shaping occurs. It is also desirable to provide a shaping tool which minimises the risk of cross contamination between patients. Additionally, it is desirable to provide a shaping tool which minimises the risks of pulmonary embolisms.

Thus, according to a first aspect of the present invention there is provided a single-use surgical shaping tool comprising: a body having a leading end and a drive end; a debris collecting store; at least one cutting means; and at least one means for directing debris into the store.

The shaping tool of the present invention is particularly suitable for removing hard issue. It will be understood that "hard tissue" as used herein is reference to any hard tissues such as enamel, cementum, dentin, bone and cartilage.

The surgical shaping tool is preferably a rasp.

As the shaping tool of the present invention is "single use", it is designed to be used on only one patient in only a single operation before being disposed of. However, it may be used to shape more than once on the same patient.

The body may be of any suitable shape depending on its end use. In one arrangement, the body is preferably an elongate body. Similarly any suitable size of body may be used. In general, the selection of the shape and dimensions of the shaping tool will depend on the intended use of the rasp.

In one arrangement, the body of the tool is an elongate body and the cross-section of the body may reduce from the drive end to the leading end such that it tapers.

In a preferred arrangement, the debris collecting store may be a cavity in the body. The cavity may be sized such that the totality of debris collected during the smoothing procedure can be stored within the body. As the shaping tool is single-use, in this arrangement, it may be simply disposed of once the surgeon has completed the procedure and thus the debris will be simultaneously disposed of with the tool.

In one alternative arrangement the body may include means for enabling collected debris to be emptied from the debris collecting store. In an arrangement in which the cavity is large enough to hold the totality of debris produced during the surgical procedure, the means for emptying may be provided to enable the debris to be removed from the shaping tool and disposed of separately. However, it will be understood that the debris collecting store may be of smaller dimensions and be emptied periodically during surgery. Any suitable means for emptying the store may be provided. For example a portion of the body may be separable or a hinged or otherwise connected door to the debris collecting store may be provided.

In an alternative arrangement, the debris collecting store may be at least partially open such that it is in communication with the external surface of the body. This will enable the collected debris to be simply removed.

The cutting means may comprise any suitable means for detaching unwanted tissue from the operation site and removing it such that when the procedure is completed substantially all unwanted tissue fragments and debris are removed.

Where more than one cutting means is present, they may be the same or different.

The or each cutting means may be a tooth. The or each tooth may be of any suitable arrangement. The or each cutting tooth may extend from the body or may be formed by a portion of the body being cut away. In an arrangement where, for example the body is of a tapered configuration the upper edge of the cut-away portion will act as a tooth. In any event, whichever arrangement is used, when the body of the shaping tool is placed against the face of the operation site and moved, the at least one cutting means cuts away a portion of the tissue, such as hard tissue, which is to be removed.

In an arrangement in which the at least one cutting means is a tooth or teeth extending outwardly from the body, they may extend at any suitable angle. In one embodiment, they may extend from the body at an angle which is acute to the leading end of the longitudinal axis of the body. In one alternative arrangement, the cutting tooth or teeth may extend from the body at an angle which is obtuse to the leading end of the longitudinal axis of the body. In a further alternative, they may extend perpendicularly to the longitudinal axis of the body.

The angle of the tooth or teeth will proscribe whether in which direction of movement cutting of the tissue occurs. For example, where the tool of the present invention is to be used to shape a bore in a bone, if the angle is acute with respect to the leading end of the longitudinal axis of the body, then removal of the hard tissue will occur when the shaping tool travels into the bore. Conversely, if the angle is obtuse with respect to the leading end of the longitudinal axis of the body, cutting will occur when the shaping tool travels outwardly from the bore.

The angle at which the at least one cutting means extends from the body may be by up to 175°, more preferably by up to 125°, still more preferably by up to 75° and most preferably by up to 35°.

The at least one cutting means is preferably located adjacent to the at least one means for directing the debris into the store. Where more than one cutting means is present there may be the same number or a different number of means for directing the debris into the store as required.

In one arrangement, the means for directing debris into the store may simply be one or more apertures in the body. In one arrangement, each cutting means is a tooth and each means for directing debris is an aperture. There may be one aperture provided for each tooth and they will generally be located adjacent to each other such that in use debris cut by a tooth is immediately passed through an aperture and into the store. The teeth may serve to not only cut tissue from the face of the site to be shaped but also to direct tissue fragments into the store by deflecting detached tissue fragments through the aperture.

The cutting means and directing means may be configured in any way which enables the optimal removal of tissue from the site to be shaped without generating excessive stresses within the hard tissue. Thus, the problems associated with the impaction of tissue debris into the face of the operation site are avoided.

In a preferred embodiment, the directing means are circular. In this arrangement, a cutting means may be provided as a tooth forming a partial cup around a portion of the aperture such that once cutting has occurred the debris falls into the cup and is directed into the store.

However, apertures which are generally rectangular may also be employed. For example, the aperture may take the form of an elongate rectangular slot which extends around part of the body of the rasp. With such a slot, the cutting means may extend from one of the longer sides of the slot, or in one alternative, extend from the body such that the cutting edge of the cutting teeth forms one of the longer sides of the perimeter of the aperture.

Where a slot is present it may be of any length which is suitable to remove hard tissue from the operation site. In a preferred embodiment, the slot is rectangular and its length extends around from about 5% to about 95%, preferably from about 15% to about 60%, more preferably from about 20% to about 40% of the perimeter of the body.

Regardless of the shape and size of the aperture, in a preferred embodiment, the angle at which the cutting means extend from the body may be adjustable. In one arrangement, the adjustment may be manually controlled by the surgeon. In one alternative, the cutting means may be formed in such a way that when used, the angle at which the cutting means extend from the body is adjusted automatically. One example of this is an arrangement in which the at least one cutting means are hinged to the body.

In this arrangement, when the shaping tool travels in the direction in which cutting occurs, the at least one cutting means extends at an angle at which cutting can occur. However, when the shaping tool travels in the opposite direction, the angle at which the at least one cutting means extends from the body is reduced to facilitate the movement of the shaping tool in that direction. In one arrangement, the angle of extension may be reduced to substantially 0° such that the cutting means lies flat against the body. In one arrangement, as the at least one cutting means collapses it covers the at least one means for directing debris. This offers an added benefit of preventing debris collected in the store from escaping to the operation site.

In a preferred embodiment of the present invention, the shaping tool is provided with flushing means to remove debris collecting in the store during use. Any suitable flushing means may be provided. In one arrangement, a liquid, such as water, which may be pressurised, is passed through the store to flush out the debris. The liquid may additionally pass through the deflecting means to communicate with an external surface of the body and serve to lubricate the operation site. This will have the added benefit of providing cooling and thereby address the problems associated with prior art devices where heating led to the aforementioned problems.

The surgical tool of the present invention may be formed of any suitable material. In one arrangement it may be formed of plastics material. In another arrangement, it may be formed of metal. In a further embodiment it may be formed of plastics and metal. Where a metal is used it may be carbon steel.

Whilst the shaping tool of the present invention may be used manually, which may require the surgeon to apply a mallet, it is preferred that the rasp is provided with driving means such as a piezoelectric driver. It will be understood that in such arrangements means for connecting the rasp to the driver will be provided and that these will generally be located at the drive end.

According to a second aspect of the present invention, there is provided a shaping tool driver comprising:
a housing;
piezoelectric drive means;
a control unit; and
shaping tool attachment means;
wherein the shaping tool attachment means is coupled to the piezoelectric drive means such that when in use energy is applied to the piezoelectric drive means, kinetic energy is transferred to the shaping tool via the attachment means.

Any suitable piezoelectric drive means may be used. The piezoelectric drive means preferably comprises a ceramic plate stack and a retainer. In a preferred embodiment, the ceramic plate stack comprises a plurality of ceramic plates. The ceramic plates preferably have a thickness of from about 0.01mm to about 1mm. The ceramic plates may be separated by thin metallic electrodes.

As will be understood piezoelectric materials have the property of producing electrical energy when mechanical forces are applied. Thus electrical energy is transferred into kinetic energy, and the material transiently changes its shape.

Where the piezoelectric drive means comprises a ceramic stack, when electrical energy is applied to the stack, the height of the stack is altered and, when the electrical supply is cut off, the stack reverts to its original height. The ceramic stack is preferably housed in a retainer which prevents movement in unwanted directions and delamination of the ceramic stack. As the piezoelectric drive means is coupled to the shaping tool attachment means, when movement is produced, it is transferred to the shaping tool attachment means and thus to an attached shaping tool. The shaping tool to which the shaping tool driver is attached is preferably the shaping tool of the above first aspect.

One advantage of using a piezoelectric drive means is that the rasp can be caused to oscillate linearly by a distance which results in optimal tissue removal but which does not exert excessive forces on the hard tissue. The distance that the rasp should travel is preferably between about 0.5mm and about 1.5mm.

The force required to cut into hard tissue using a piezoelectrically-driven rasp is around 150N to 250N. This is in sharp contrast to the forces produced by the use of conventional tamps or broaches with a mallet which are in the excess of 8000N thereby increasing the risk of intra-operative fractures. The reduced force associated with the use of a piezoelectric driver means that the heat generated is substantially reduced such that the risk of damage to tissue adjacent to the bore is minimised.

A further advantage associated with piezoelectric drive means is their low energy consumption. In a preferred embodiment of the present invention, the energy consumption of the rasp driver is less than 1600 Watts.

As the voltage and amplitude of the electrical energy supplied to the piezoelectric drive means has an effect on the type of motion which is produced, the control unit is preferably capable of adjusting the voltage and amplitude of the electrical energy supplied to the piezoelectric drive means.

According to a third aspect of the present invention there is provided a method of producing a single-use surgical shaping tool comprising the steps of:
a) preparing a generally flat shaping tool blank;
b) forming at least one semi-shear in the blank;
c) folding the blank into a shaping tool;
d) joining the meeting edges to form a continuous surface; and
e) generating at least one cutting means from the at least one semi-shear.

The semi-shear is preferably formed using a forming tool with a punch and die. In a preferred embodiment, the shaping tool is the shaping tool of the above first aspect.

In a preferred embodiment of the present invention, the cutting means is generated by the folded blank being gripped by gripping means and moved against a grinding wheel. The gripping means is preferably a robotic arm.

Once the cutting means has been formed, the rasp is preferably finished to remove any burs or loose material which may be present on the cutting means.

The hard tissue may be the walls of a bore.

The apparatus of the present invention can be used in a method of shaping hard tissue, the method comprising:
a) accessing the hard tissue; and
b) using a shaping tool to shape the hard tissue as desired
wherein the shaping tool is a single-use surgical shaping tissue according to the first aspect of the present invention.

The hard tissue will generally be shaped to receive a prosthetic implant. The method of the present invention may enable the tissue to be shaped to a tolerance of ±0.1 mm the three dimensional form of the prosthesis to be implanted.

The shaping tool may also include barbs which retain and break up bone swarf.

For ease of reference in the following discussion, the shaping tool will be identified as a "rasp".

The shaping tool, driver, method of shaping tool formation and method of shaping hard tissue of the present invention will now be described by way of example, with reference to the accompanying drawings in which:
- Figure 1: is a perspective view of a rasp of one embodiment of the present invention;
- Figure 2: is a side view of the rasp of Figure 1;
- Figure 3: is a rasp of a second embodiment of the present invention;
- Figure 4: is a rasp of a third embodiment of the present invention;
- Figure 5: is a rasp of a fourth embodiment of the present invention;
- Figure 6: is a rasp of a fifth embodiment of the present invention;
- Figure 7: is a rasp of a seventh embodiment of the present invention;
- Figure 8: is an exploded view of the cutting means provided on the rasp of in Figure 7;
- Figure 9: is a rasp of an eighth embodiment of the present invention;
- Figure 10: is a rasp of a ninth embodiment of the present invention in a closed position;
- Figure 11: is the rasp of Figure 10 in an open position;
- Figure 12: is a rasp of a tenth embodiment in use in the human femur;
- Figure 13: is a perspective view of a rasp of an eleventh embodiment which is suitable for use in cutting the acetabulum;
- Figure 14: is a side view of the embodiment of Figure 13;
- Figure 15: is an underside view of the embodiment of Figure 13;
- Figure 16: is an exploded view of B of Figure 15;
- Figure 17: is an extended view of the embodiment of Figure 13 with detachable drive shaft;
- Figure 18: illustrates a rasp blank;
- Figure 19: illustrates a part-formed rasp produced from the rasp blank illustrated in Figure 18;
- Figure 20: is an expanded view of components of a further arrangement.
- Figure 21: is a side view of the rasp of Figure 20.
- Figure 22: is a front view of the rasp of Figure 20.
- Figure 23: is a sectional view of the device on line A1 of Figure 22; and
- Figure 24: is an exploded view of the AK of Figures 23.

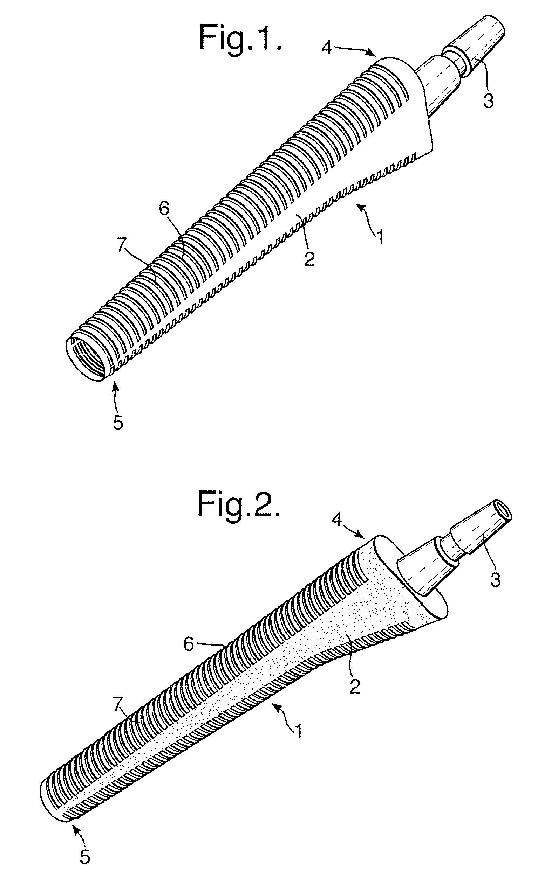

As illustrated in Figures 1 to 10, the rasp 1 of one arrangement has an elongate body 2 and rasp driver attachment means 3. The body has a driver end 4 and a leading end 5. The cutting means are in the form of cutting teeth 6 and apertures 7. In Figures 1 and 2 the apertures are elongate rectangular slots which extend around approximately 20% of the perimeter of the body 2.

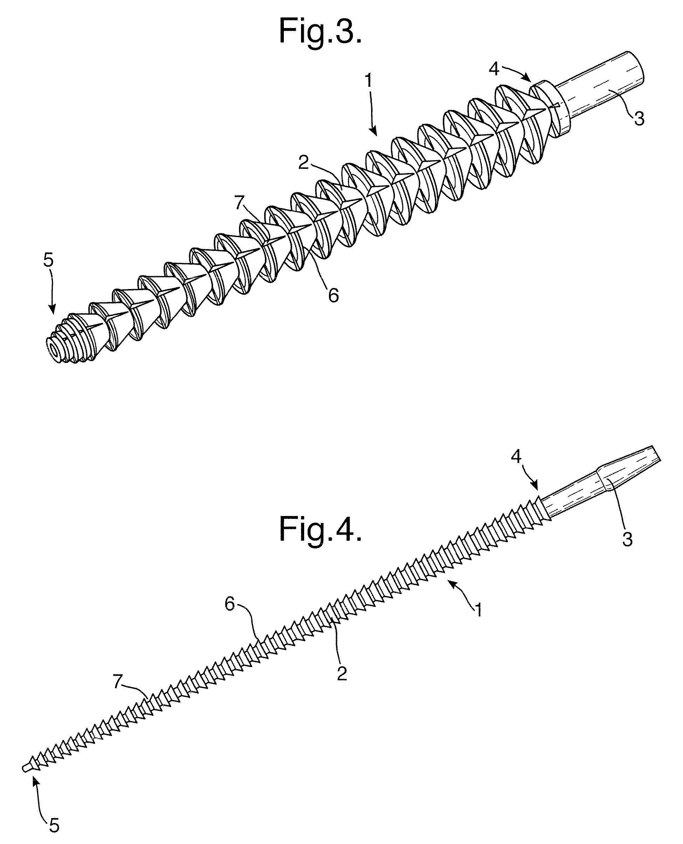

As illustrated in Figure 3, the cutting teeth 6 may be more pronounced than those illustrated in Figures 1 and 2. The cutting teeth 6 illustrated in this rasp extend from the body 2 at an angle which is acute with respect to the leading end 5 of the longitudinal axis of the body 2. Thus, in use, cutting will occur when the rasp travels into the bore. Any detached tissue will be directed by the teeth into a store (not shown) within the body.

An alternative type of rasp 1 is shown in Figure 4, where the cutting teeth 6 extend from the body 2 at an angle which is obtuse with respect to the leading end 11 of the longitudinal axis of the body 2. Thus, in use, cutting will occur when the rasp travels outwardly from the bore.

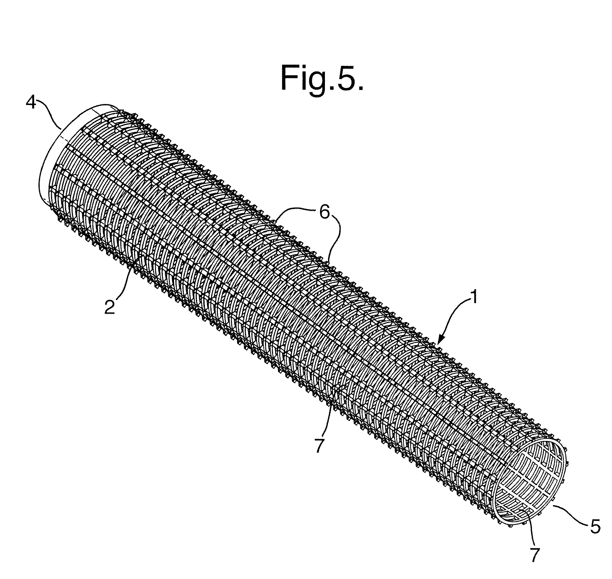

In Figure 5 a still further type of rasp 1 is illustrated. It can be seen that the apertures 7 are larger than those shown in Figures 1 to 4. As with the rasp of Figure 3, the cutting teeth 6 extend from the body 2 at an angle which is acute with respect to the leading end 5 of the longitudinal axis of the body 2, and thus, in use, cutting will occur when the rasp travels into the bore.

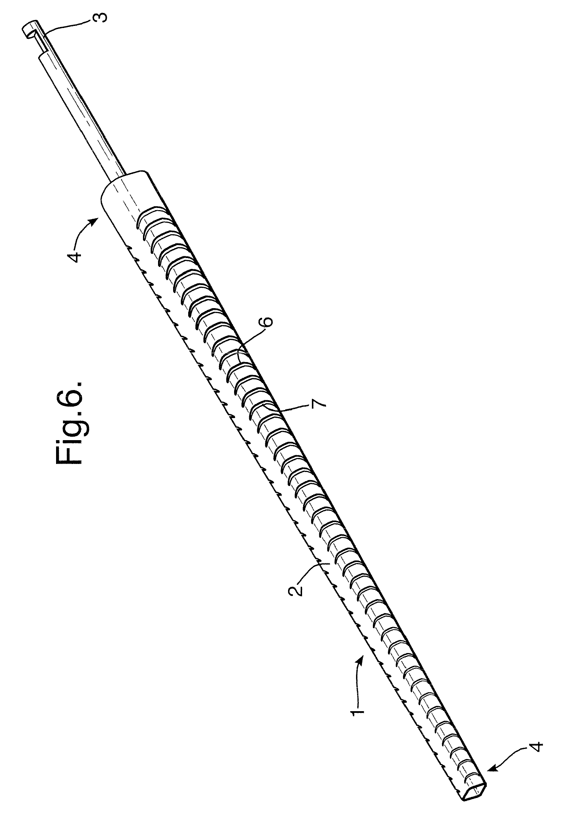

The rasp shown in Figure 6 is provided with a different type of rasp driver attachment means 3 to the rasp of Figures 1 and 2. In the illustrated arrangement the body 2 of the rasp 1 has a reduced cross-section than those illustrated previously and thus, this rasp is particularly suitable for use where the bores to be created are small.

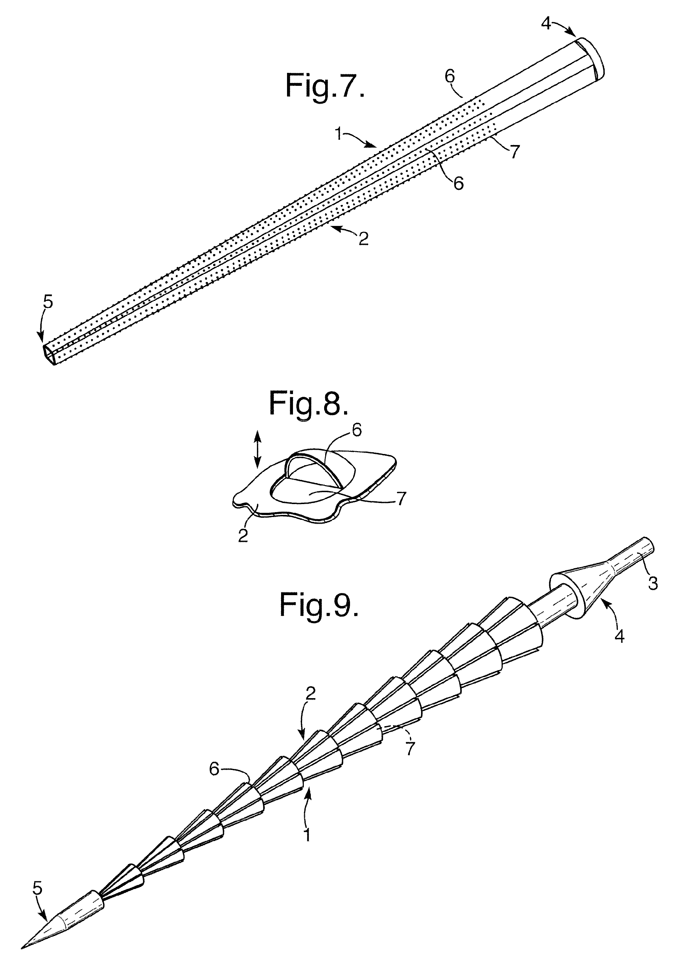

The rasp 1 shown in Figure 7 is provided with cutting teeth 6 which are attached to the body 2 by a hinge. This allows the cutting teeth 6 to cut tissue and deflect it through the apertures 5 into the cavity when the rasp 1 travels into the bore. However, when the rasp 1 travels out of the bore, the cutting teeth 6, upon contact with the face of the bore, are pressed down until their angle of protrusion from the body 2 is approximately zero, and the apertures 7 are completely covered. This prevents any tissue fragments from escaping out of the cavity into the bore. An exploded view of the hinged cutting tooth 6 and aperture 7 is shown in Figure 8. It will be understood that the hinged tooth may be arranged such that it is collapsed on insertion of the rasp into the bore and opens to allow cutting as the rasp is withdrawn from the bore.

Figure 9 illustrates a rasp 1 having elongate cutting teeth which extend from the body 2 at an angle which is obtuse with respect to the leading end 5 of the longitudinal axis of the body 2. Cutting occurs when the rasp 1 travels out of the bore.

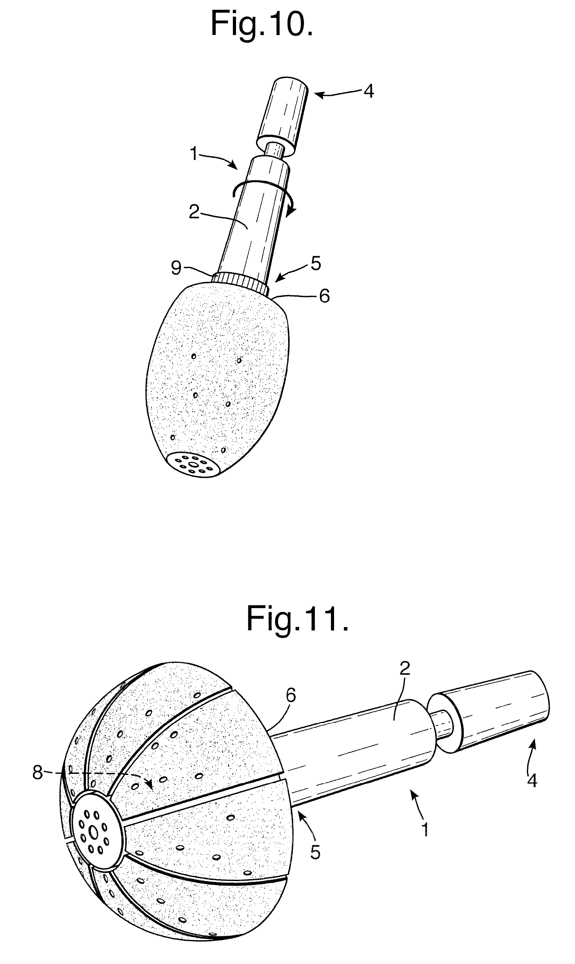

Figure 10 shows a rasp 1 which comprises an alternative type of cutting means. The rasp 1 is provided with only one cutting tooth 6 connected to the leading end 5 of the body 2. In use, the rasp 1 is placed into a bore and then the body 2 is rotated to cause the cutting tooth 6 to expand to provide cavity 8 which is external from the body 2. Figure 11 illustrates the rasp 1 shown in Figure 10 when fully opened. The body 2 is provided with graduations 9 to assist the surgeon with rotating the body 2. Once in a fully opened position, the rasp 1 is withdrawn from the bore and the tooth engages and cuts the tissue, deflecting it into external cavity 8.

The rasps as illustrated in Figures 1 to 11 are particularly suitable for use in a bore such as in the intramedullary canal in the femur.

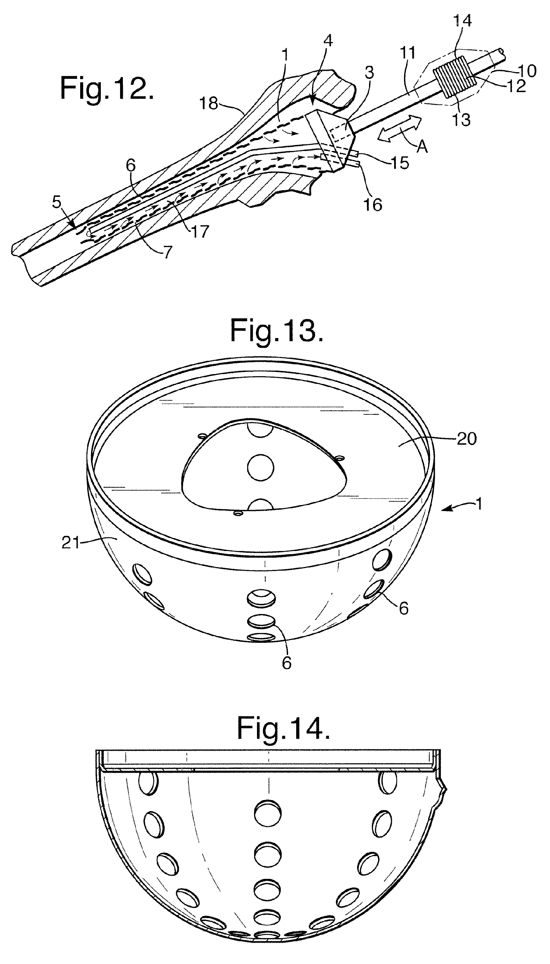

Figure 12 illustrates how a rasp of the present invention may be used with the rasp driver of the present invention to prepare a bore in the femur 18. Rasp 1 is coupled to rasp driver 10 by means of the rasp driver attachment means 3. The rasp attachment means 3 co-operates with the shaft 11 of the rasp driver 10. The ceramic stack 12 has electrical energy applied to it intermittently via thin metal electrodes 13 which are placed between the ceramic disks 14. This causes the ceramic stack to expand and contract in the direction illustrated by arrow A. This movement is transferred via shaft 11 and rasp driver attachment means 3 to the rasp 1.

The cutting teeth 6 of rasp 1 extend externally from the body 2 at an angle which is acute with respect to the operative end 5 of the longitudinal axis of the body 2. This means that every expansion of the ceramic stack which drives the rasp 1 into the bore results in the face of the bore being cut by the cutting teeth 6. The tissue which is detached by this action is deflected by the cutting teeth 6 through apertures 7 into the store 17 of the body 2.

Water, or any other inert liquid, may be fed under pressure via fluid inlet tube 15 into the store 17 of the body 2 where it emerges near the operative end 5 of the body 2. The liquid then travels from the operative end 5 of the store 17 to the driver end 4 where it exits via fluid exit 16. As the fluid moves through the cavity 17, it carries the removed tissue which is collected within the cavity 17 away, which prevents rasp 1 from becoming clogged with such debris.

When a rasp 1 and rasp driver 10 are used in the manner illustrated in Figure 9 a tissue removal rate of up to 10 grams per second may be attained.

An alternative surgical tool/rasp of the present invention is illustrated in Figures 13 to 17. This tool is particularly suitable for shaping the acetabulum prior to the insertion of a prosthetic acetabular cup. The rasp 1 includes a plurality of cutting teeth 6. The substantially hemispherical rasp surface is provided with a drive plate 20 which may be attached to the cutting surface 21 by any suitable means. In one arrangement, where both components are made of metal, the components may be welded together.

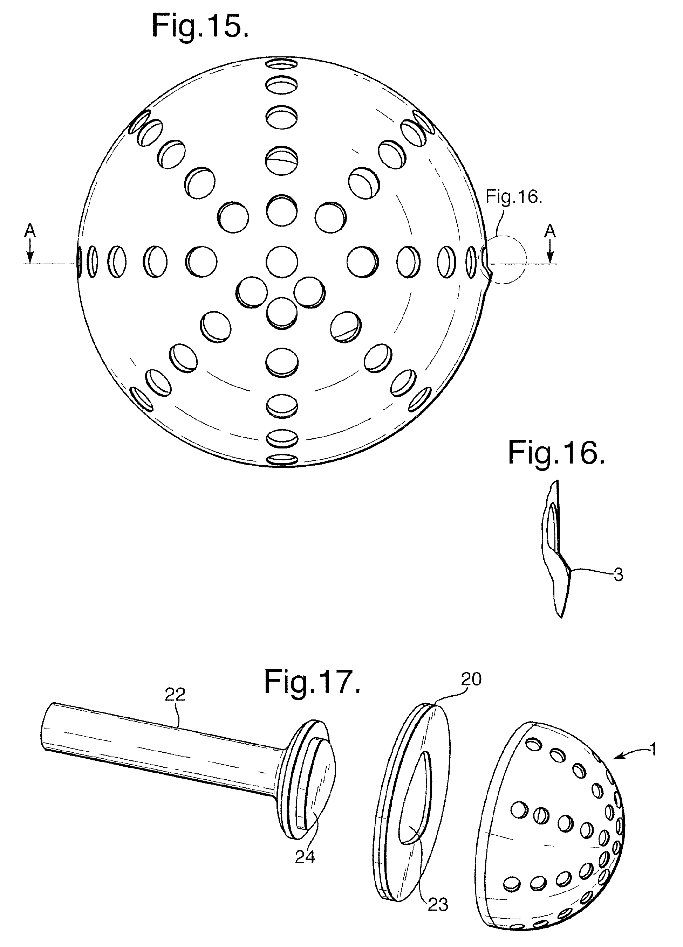

The teeth 6 are formed from a hole having an indented profile. The holes are positioned in such a manner as to overlap thus forming a spherical cut with minimum striations. One suitable arrangement of holes is illustrated in Figure 15 although it will be appreciated that other arrangements, such as a spiral arrangement, may be appropriate.

A drive shaft 22 may be provided which will contain means which will enable the drive shaft to interconnect with the drive plate 20 to attach the rasp 1 to the drive shaft 22. It will be understood that the rasp portion will be sized depending on the acetabular prosthesis to be inserted. However, a single sized drive shaft may be used for all acetabular sizes. In one arrangement, the drive shaft may be nondisposable but will be able to withstand sterilisation.

The rasp of this embodiment may collect the debris inside the hemnespherical cup. It can then be recovered, if required, through the aperture 23 in the drive plate. It will be understood that in use, the aperture 23 is blocked by the interlocking feature on the drive shaft 22 which as illustrated in Figure 17 may be a tri-lobed component. The drive shaft may be a quick release on the drive plate.

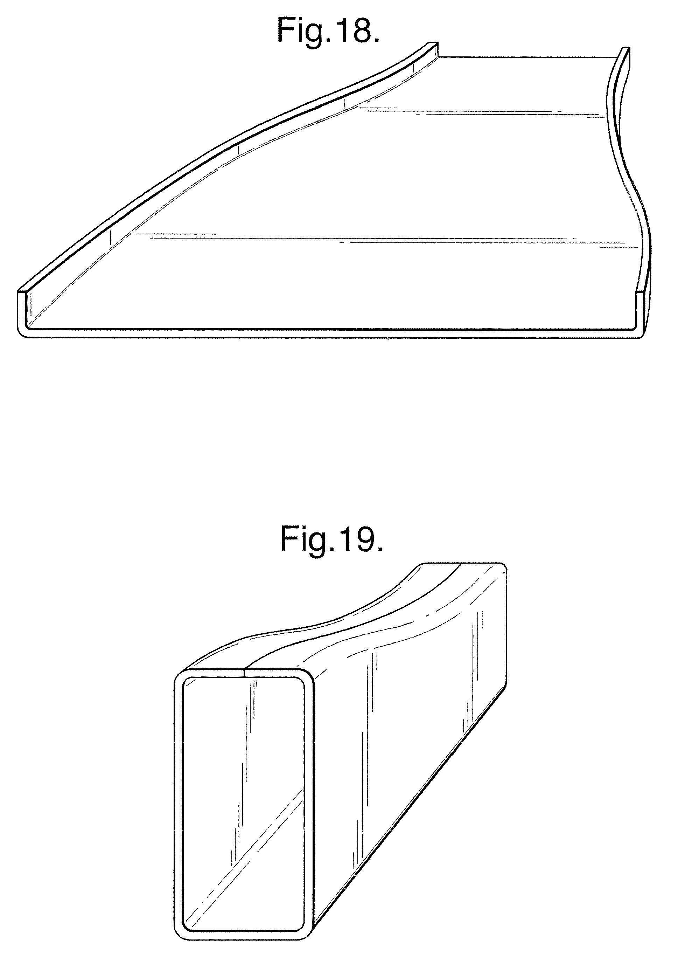

Fig 18 illustrates a rasp blank which has been partially folded. The semi shears have not yet been pressed into the surface of the blank. Once this has been done, the blank will be folded up to form the basic shape of the rasp.

Fig 19 illustrates a rasp blank which has been folded to form the basic rasp shape, and which has also had its joining edges joined to form a continuous surface. The next step would be to form cutting means from the semi shears, and this may be done using a robotic arm to grip the rasp to ensure the cutting means are formed to the exact requirements of the rasp.

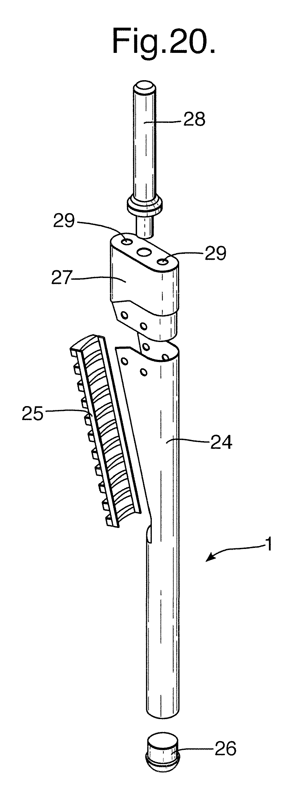

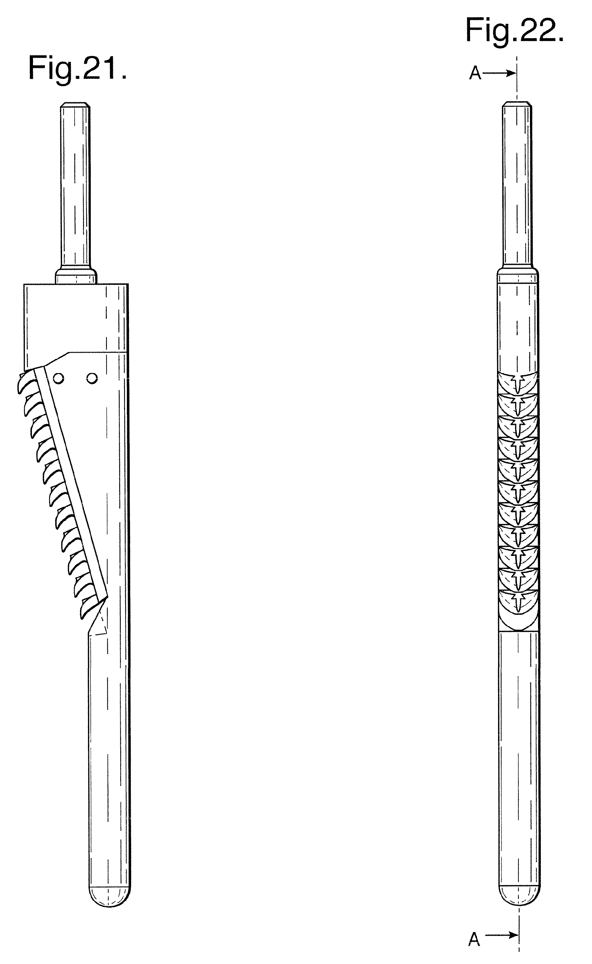

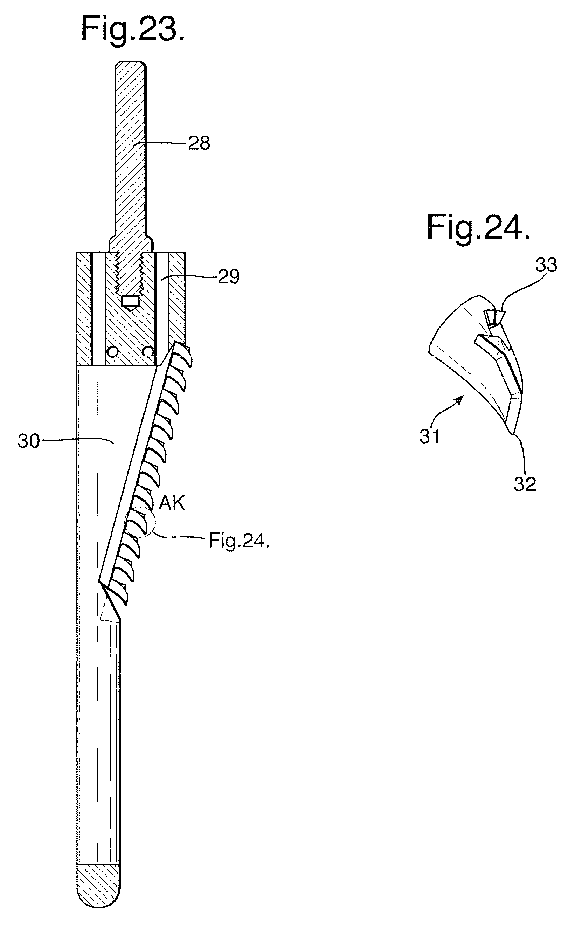

A still further arrangement is illustrated in Figures 20 to 24. In this embodiment the rasp 1 comprises a body 24, the hollow structure of which enables retention of bone debris and which includes a void space 30. The rasp additionally includes a rasp cutting edge 25. The bone is closed at the distal end by a nose piece 26.

At the proximinal end, the rasp is closed by a connection block 27 which has three apertures therethrough. A drive shaft 28 will be placed in one aperture. The drive shaft may be coupled to a hammer or reciprocating shaft. The other apertures 29 can be used for saline flushing.

The rasp includes teeth 31 which have a cutting edge 31. Barbs 33 to retain and break up bone swarf are also provided.

## Claims

1. A single-use surgical shaping tool comprising: a body having a leading end and a drive end; a debris collecting store; at least one cutting means; and at least one means for directing debris into the store.

2. A single-use surgical shaping tool according to Claim 1 wherein the body is an elongate body.

3. A single-use surgical shaping tool according to Claim 1 or 2 wherein the cross-section of the body reduces from the drive end to the leading end such that it tapers.

4. A single-use surgical shaping tool according to any one of Claims 1 to 3 wherein the debris collecting store is a cavity in the body.

5. A single-use surgical shaping tool according to Claim 4 wherein the cavity is sized such that the totality of debris collected during the procedure can be stored.

6. A single-use surgical shaping tool according to Claim 4 or 5 wherein the body includes means for enabling the debris to be emptied from the cavity.

7. A single-use surgical shaping tool according to any one of Claims 4 to 6 wherein the cavity is not closed such that it is in communication with the external surface of the body such that debris may be removed.

8. A single-use surgical shaping tool according to any one of Claims 1 to 7 wherein more than one cutting means is present.

9. A single-use surgical shaping tool according to any one of Claims 1 to 8 wherein the or each cutting means is a tooth.

10. A single-use surgical shaping tool according to Claim 9 wherein the or each tooth extends from the body.

11. A single-use surgical shaping tool according to Claim 9 wherein the or each tooth is formed by a portion of the body being cut away.

12. A single-use surgical shaping tool according to any one of Claims 9 to 11 wherein the or each tooth extends from the body at an angle which is acute to the leading end of the longitudinal axis of the body.

13. A single-use surgical shaping tool according to any one of Claims 9 to 11 wherein the or each tooth extends from the body at an angle which is obtuse to the leading end of the longitudinal axis of the body.

14. A single-use shaping tool according to any one of Claims 1 to 13 wherein the angle at which the or each cutting means extends from the body is adjustable.

15. A single-use surgical shaping tool according to any one of Claims 1 to 14 wherein the or each cutting means is located adjacent to the at least one means for directing the debris into the store.

16. A single-use surgical shaping tool according to any one of Claims 1 to 15 wherein the means for directing debris into the store is one or more apertures in the body.

17. A single-use surgical shaping tool according to any one of Claims 1 to 16 additionally provided with flushing means to remove debris collecting in the store during use.

18. A single-use surgical shaping tool according to Claim 17 wherein the flushing means is provided by liquid passed through the store.

19. A single-use surgical shaping tool according to any one of Claims 1 to 16 wherein the tool is provided with a piezoelectric driver.

20. A shaping tool driver comprising:
a housing;
piezoelectric drive means;
a control unit; and
shaping tool attachment means;
wherein the shaping tool attachment means is coupled to the piezoelectric drive means such that when in use energy is applied to the piezoelectric drive means, kinetic energy is transferred to the shaping tool via the attachment means.

21. A shaping tool driver according to Claim 20 wherein the piezoelectric drive means comprises a ceramic plate stack and a retainer.

22. A shaping tool driver according to Claim 21 wherein the ceramic plates have a thickness of from about 0.01mm to about 1mm.

23. A shaping tool driver according to Claim 21 or 22 wherein the ceramic plates are separated by thin metallic electrodes.

24. A shaping tool drive according to any one of Claims 20 to 23 in combination with the shaping tool of any one of Claims 1 to 19.

25. A method of producing a single-use surgical shaping tool comprising the steps of:
a) preparing a generally flat shaping tool blank;
b) forming at least one semi-shear in the blank;
c) folding the blank into a shaping tool;
d) joining the meeting edges to form a continuous surface; and
e) generating at least one cutting means from the at least one semi-shear.
